# EUROPEAN PATENT SPECIFICATION

(11) **EP 0 382 812 B1**
(45) Date of publication and mention of the grant of the patent: **03.11.1993**
(21) Application number: 89907494.2
(22) Date of filing: 02.06.1989
(51) Int. Cl.: A01N 43/08

(54) **A NEW INDUSTRIAL ANTIMICROBIAL: USES FOR 2-(2-BROMO-2-NITROETHENYL)-FURAN AND A NEW PROCESS OF FORMING 2-(2-BROMO-2-NITROETHENYL)-FURAN**
INDUSTRIELLES ANTIMIKROBIELLES MITTEL: VERWENDUNGEN FÜR 2-(2-BROM-2-NITROÄTHENYL)-FURAN UND EIN HERSTELLUNGSVERFAHREN FÜR 2-(2-BROM-2-NITROÄTHENYL)-FURAN
NOUVELLE PREPARATION ANTIMICROBIENNE: UTILISATION POUR 2-(2-BROMO-2-NITROETHENYLE)-FURANE ET NOUVEAU PROCESSUS DE FORMATION DE 2-(2-BROMO-2-NITROETHENYLE)-FURANE

(30) Priority: 10.06.1988 US 205078
(43) Date of publication of application: 22.08.1990
(73) Proprietor: GREAT LAKES CHEMICAL CORPORATION, West Lafayette Indiana 47906 (US)
(72) Inventor: McCOY, William, F., West Lafayette, IN 47906 (US); THORNBURGH, Scott, West Lafayette, IN 47906 (US)
(74) Representative: Bond, Bentley George
(86) International application number: US8902417
(87) International publication number: WO8911793

(56) References cited:
- JP-A- 6 161 764
- US-A- 2 335 384
- US-A- 3 629 465
- L.F. FIESER et al.: "Organische Chemie", 2nd edition, 1968, Verlag-Chemie, Weinheim, DE
- Potemin Khim. Farm. Zb, vol. 6, 1972; Z.N. NAZAROVA et al.: "Synthesis of Some Furylnitroolefins with Potential Biological Activity" pp. 692-632
- Zhurnal Organicheskoi Khimii, vol. 8, 1972; M.G. GRUNTFEST et al.: "Physiochemical Properties and Reactivities of Furylnitroolefins" pp. 405-411
- Zhur. Cebshcksi Khim., vol. 24, 1954; Z.N. NAZAROVA: "Concerning B-Nitrovinyl-5-Substituted Furans" pp. 589-592
- Doklady Akademiy Nauk SSSR, vol. 232, 1977; G.L. KAMALOV et al.: "Study of the Formation and Properties of a Series of Furyl-Substituted Ethylenes" pp. 68-71
- Coll Czech. Chem. Comm., vol. 49, 1984; R. KADA et al.: "Preparation of 5-x-2-(2-Nitrovinyl)-furans and their Reaction with Nucleophiles" pp. 2496-2501

## Description

This invention relates to the use of 2-(2-bromo-2-nitroethenyl)-furan (BNEF) as a broad spectrum antimicrobial agent.

A number of industrial organo-bromine nonoxidizing antimicrobials, including β-bromo-β-nitro-styrene, are known. Nonoxidizing antimicrobials are used in many industrial systems in which strong oxidizing antimicrobials such as chlorine or bromine cannot be used. Industrial systems generally requiring nonoxidizing antimicrobials include water systems, pulp and paper manufacturing, metal working fluid preservation, latex paint preservation, wood preservation, cosmetics preservation, oil field and institutional hard-surface disinfection. However, despite the many commercially available nonoxidizing antimicrobials, none are entirely suitable for every application due to efficacy, safety, environmental acceptability and cost.

The antimicrobial activity of various 2-furylethylenes, such as 2-(2-nitroethenyl)-furan is also yell known. The literature suggests that the microbiocidal activity of the nitroethenylfurans is not sufficient for industrial antimicrobial application unless the furan ring has a 5-nitro substitution. Unfortunately, 5-nitro substituted furan antimicrobials are mutagenic and, in some cases, even carcinogenic in rodents: therefore, 5-nitrofuran derivatives are unsuitable for industrial applications. However, the prior art has failed to recognize the effective antimicrobial activity of 2-(2-bromo-2-nitroethenyl)-furan (BNEF) as an nonoxidizing antimicrobial.

Russian Patent 1,027,663, issued January 1, 1967, discloses the usage of furylnitroalkylenes as antimicrobials. The furylnitroalkyenes are applied to the root zone of a plant or to the soil to control nematodes.

Nazarova and Potemkin in an article entitled "Synthesis of Some Furylnitroolefins with Potential Biological Activity" describe the laboratory preparation of selected furylnitroolefins including BNEF. The process used to prepare BNEF utilizes an 100 percent stoichiometric excess of aqueous potassium hydroxide as a catalytic agent. However, it has been found that the BNEF formed by the described process is undesirably contaminated with nonbrominated nitroethenylfuran, a compound with lower antimicrobial activity.

The Russian article "Physiochemical Properties and Reactivities of Furylnitroolefins" by Gruntfest et al., discloses the physiochemical properties, structures and reactivities of a series of furylnitroolefins. The article fails to disclose the antimicrobial activities of any of the studied furylnitroolefins.

Japanese Patent 59/184463 discloses a antimicrobial use of nitroethenylfuran used as a component of an antifouling coating for ship bottoms.

Accordingly, a primary object of the present invention is the development of a new broad spectrum industrial antimicrobial agent incorporating 2-(2-bromo-2-nitroethenyl)-furan.

Another object is to obtain a new broad spectrum antimicrobial agent of the character described that is nonoxidizing.

The foregoing and other objects, advantages and features of the present invention may be achieved by using 2-(2-bromo-2-nitroethenyl)-furan as a broad spectrum industrial antimicrobial agent. More particularly, it has been found that 2-(2-bromo-2-nitroethenyl)-furan can be used in water treatment, pulp and paper manufacturing, metal working fluids, and various other nonoxidizing biocides applications. 2-(2-bromo-2-nitroethenyl)-furan is effective against a wide spectrum of bacteria, algae, and fungi.

Figure 1 is a graph showing the relationship between bacterial viability and time in the usage of 2-(2-bromo-2-nitroethenyl)-furan against the most common bacterium found in industrial cooling water.

Figure 2 is a compilation of results from the ASTM E600-85 Pulp and Paper efficacy test.

Figure 3 is a graph showing the relationship between bacterial viability and concentration of 2-(2-bromo-2-nitroethenyl)-furan in the three major categories of metal working fluid.

Figure 4 is a graph showing the efficacy of 2-(2-bromo-2-nitroethenyl)-furan in industrial cooling water applications according to the ASTM E-645-85 method.

Figure 5 is a graph showing the kinetics of disinfection of 2-(2-bromo-2-nitroethenyl)-furan in comparison to its styrene analogue.

The present invention relates to new efficacous uses of 2-(2-bromo-2-nitroethenyl)-furan (BNEF) as a versatile chemical antimicrobial agent. BNEF has been shown to be an extremely powerful antimicrobial agent. It is active at low levels against a broad spectrum of microorganisms including bacteria, such as Gram negative bacteria such as Pseudomonas aeruginosa Xanthomonas prunii, Erwina anylovora, or Escherichia coli, and Gram positive bacteria such as Bacillus megaterium, Streotoccus pyogenes, Clostridium botulium, or Staohylococcus aureus: algae such as Chlorella pyrenoidosa and other green algae, and blue-green algae; and fungi such as Asoergillus niger, Trichomonas vaginitis, Alternaria solani, Trichoderma, Saccharomyces, and Rhizoctania. BNEF can be produced from furfural, an inexpensive biodegradable agricultural waste product. Further, BNEF is a strong chromophore and can therefore easily be detected.

The invention also relates to a novel antimicrobial composition. The antimicrobial composition which exists in liquid form comprises a mixture of BNEF and an inert carrier selected from, tetrahydrofuran, tetrahydrofurfuryl alcohol, N-methylpyrrolidone, and dimethylformamide. Tetrahydrofurfuryl alcohol has been found to be a particularly useful carrier. The mixture comprises 0.1-50% w/v of BNEF and 50-99.9% w/v of carrier. More desirably, the composition comprises 1-50% w/v of BNEF and 50-99% w/v of the carrier. Most desirably, the composition comprises 20% w/v of BNEF and 80% w/v of carrier.

In addition, the invention relates to a method of inhibiting microbial growth in an aqueous media by the addition of BNEF. Possible aqueous media include cooling water, pulp and paper making process flows, metal working fluids, air-washers, oilfield injection water and drilling muds, acrylic latex paint emulsions, adhesives and coatings, swimming pools and spas, and cosmetics. An antimicrobially effective amount of BNEF is added to the aqueous media. An antimicrobially effective amount can be found in the range of approximately 0.5 parts per million (ppm) to 300 ppm.

In general, BNEF is a broad spectrum antimicrobial with a multitude of possible applications. The possible applications can be divided into four groups; nonoxidizing antimicrobial applications; the biocidal applications; applications as a synergist with both other nonoxidizing biocides and oxidizing biocides; and other novel applications. Possible nonoxidizing antimicrobial applications include but are not limited to: water treatment, pulp and paper manufacturing, metal working fluid preservation, fuel preservation, latex paint preservation, cosmetics preservation, and swimming pool and spas applications. BNEF can be used in biocidal applications for mollusks such as Corbicula (clams) and Oncomelania bulinus (snails); for protozoa such as Giardia and Entamoeba, and for fungi such as Trichomonas vainalis. Further, BNEF can be used in combination with other nonoxidizing biocides such as, for example, quarternary ammonium salts, isothiazolone biocides, dibromonitrilopropionamide (DBNPA), 2-bromo-2-nitro-propane-1,3-diol, tributyltin oxide, triazine herbicides, 3,3,4,4-tetra-chloroterahydrothiophene 1,1-dioxide, methylene bisthiocyanate, or with oxidizing biocides such as: chlorinated isocyanurates, halogenated hydantoins, hypochlorite liquids and solids, chlorine dioxide, hydrogen peroxide, peracetic acid, and ozone.

### EXPERIMENTAL EVALUATIONS

It has been found that BNEF is active against a broad spectrum of microorganisms. Table 1 shows the minimum inhibitory concentrations against bacteria and algae for a number of biocidal compounds including BNEF. The data illustrates BNEF is active at very low concentrations.

**Table 1**

| Efficacy of 2-(2-bromo-2-nitroethenyl)-furan and related compounds against bacteria and algae in laboratory tests. | | | |
|---|---|---|---|
| Compounds | Minimum Inhibitory Conc (ppm) | | |
| | Pseudomonas aeruginosa | Baccilus megaterium | Chlorella pyrenoidosa |
| furylethylene | ≧1000 | ≧1000 | 250-1000 |
| 3-(2-furyl)acrolein | 250-500 | 250-500 | 16-63 |
| 2-nitro-1-(2-furyl)ethylene | 63-125 | 63-125 | 16-63 |
| 2-(2-bromo-2-nitroethenyl)furan | 5-10 | 5-10 | 4-16 |
| 5-nitro-2-furaldehyde | 6-12 | 3-6 | 4-16 |

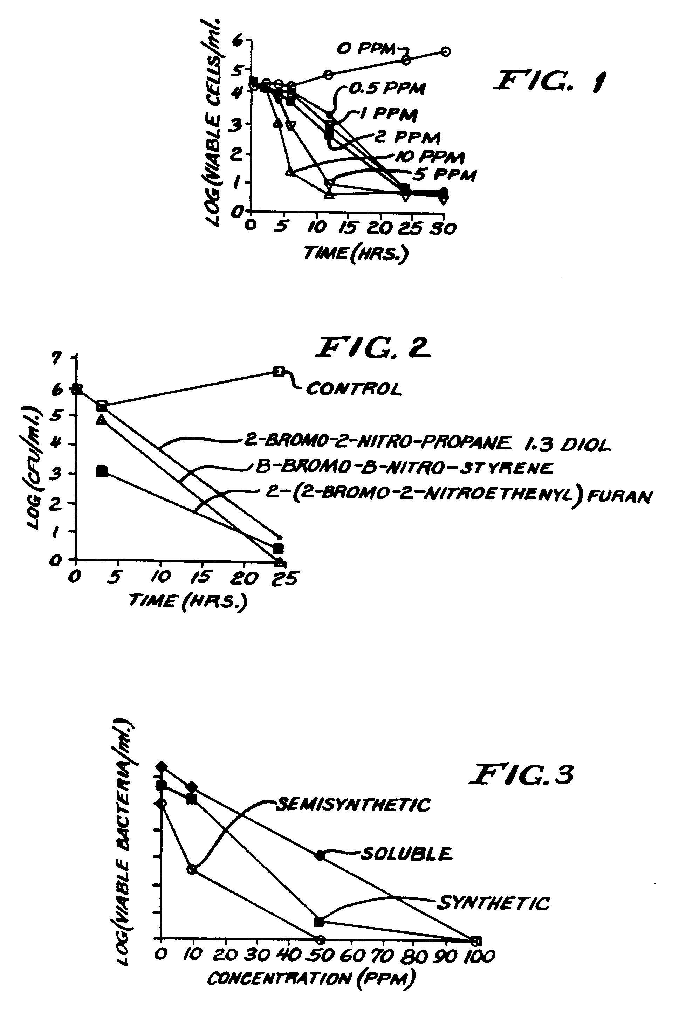

Figure 1 illustrates a decrease in the viability of Pseudomonas aeruginosa, a common bacterial genus found in industrial cooling water upon contact with different concentrations of BNEF. The experiment was performed in phosphate buffered water at a pH of 7.2. As seen in the Figure, the efficacy data indicate about 10 ppm of BNEF is required to eliminate 99.9% of the test inoculum after 6 hours of contact.

BNEF can also be used as an antimicrobial in pulp and paper manufacturing applications. Figure 2 shows results from the ASTM Pulp and Paper efficacy test (ASTM E600-85). This laboratory test indicates that BNEF is at least as active as two commercially successful antimicrobials currently used in this industry.

In addition to pulp and paper manufacturing, BNEF can be used as a metal working fluid preservative. Test data show BNEF is effective in three categories of metal working fluids (soluble oil, semisynthetic, and synthetic) against bacteria, as seen in Figure 3, and fungi. These tests were performed in the following manner. A typical soluble oil, semi-synthetic, and synthetic metalworking fluid were prepared with tap water at a ratio of 20:1. A field sample of "spoiled" fluid was mixed with a nutrient broth (50/50) and incubated for 24 hours to serve as the bacterial inoculum. Fungi isolated from metalworking fluids were grown on agar plates. The fungi were harvested into a suspension which then served as the fungal inoculum. At time zero, 50 ml aliquots of each fluid were prepared. The fluids were inoculated and dosed with the antimicrobial agent at different concentrations. Time zero bacterial and fungal counts were taken on each control fluid. The samples were then placed on a mechanical shaker (at room temperature) for 72 hours. Bacterial and fungal counts were then performed. The preferred level of BNEF in this application is in the range of 10 to 100 ppm.

BNEF can also be used in water treatment such as for example treatment of industrial cooling water. Figure 4 shows the efficacy of BNEF in industrial cooling water. As seen from the Figure, approximately a 99 percent reduction in viability of naturally occurring microorganism in industrial cooling water can be achieved with the application of 5 ppm of BNEF and 24 hours of contact. Shorter contact times require a higher concentration of BNEF in the application. Preferably an antimicrobially effective amount of BNEF should be added to reduce the viability of microorganisms by at least 90 percent. It is especially preferred to employ about 1 ppm to about 5 ppm of BNEF.

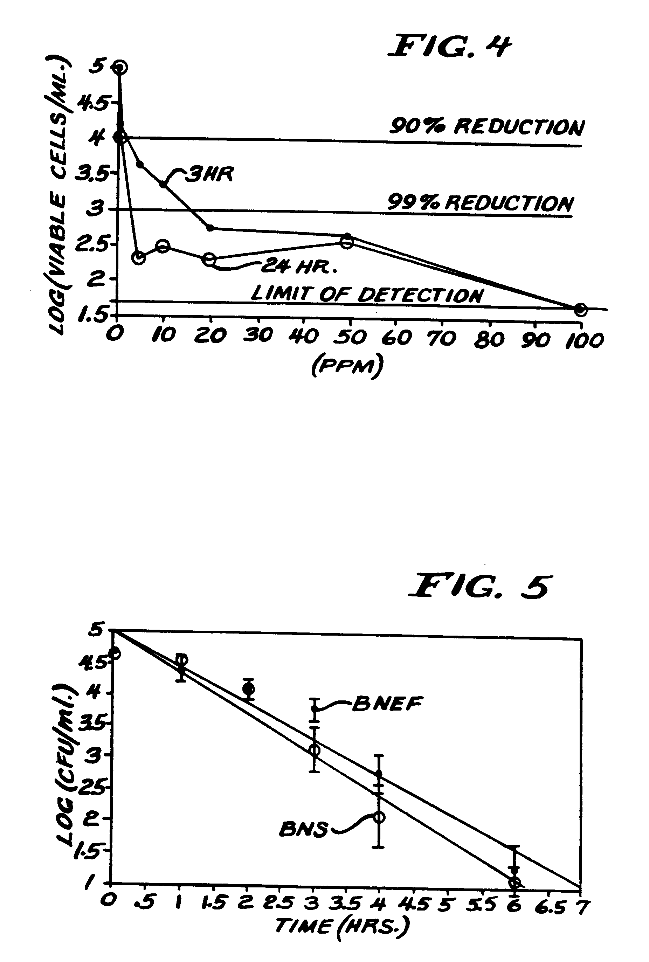

Figure 5 shows the kinetics of disinfection of 10 ppm (w/v) BNEF in comparison to its styrene analogue, B-bromo-B-nitrostyrene, a successful commercial water treatment antimicrobial in a phosphate buffer at a pH of 7.0. The data were analyzed statistically and demonstrate BNEF is as effective as an antimicrobial in water treatment application as B-bromo-B-nitrostyrene.

Virtually all modern water treatment applications are alkaline. Even in an alkaline condition, BNEF is quite active. Data in Table 2 show the contact time of BNEF required for a 99.9 reduction in viability of Pseudomonas aeruginosa in water. The data were acquired in the following manner. Several pure culture cell suspensions of Pseudomonas aeruginosa were prepared in phosphate-buffered water that had been adjusted to pH 7.0 or pH 8.1. The initial concentration of viable cells was determined by counting colony forming-units in the standard spread plate method. A tetrahydrofurfuryl alcohol (THFA) solution containing the antimicrobial (90% THFA, 10% antimicrobial) was added to cell suspensions at each pH such that 10 ppm of the antimicrobial was dosed. Similarly, THFA was added without antimicrobial to cell suspensions at each pH value to serve as no-treatment controls. The concentration of viable cells was then determined at regular time intervals for all of the cell suspensions until at least 99.9% reduction in viability (compared to the controls) was achieved by the antimicrobial treatment.

**Table 2**

| Contact time of 10 ppm of 2-(2-bromo-2-nitroethenyl)-furan required for 99.9% reduction in viability of Pseudomonas aeruginosa in water. | | |
|---|---|---|
| pH | Initial Cell Concentration | Required Contact Time (99.9% reduction) |
| 7.0 | 3.2 X 104 | 5 hrs |
| 8.1 | ∼1.0 X 104 | 1 hrs |

The data in Table 2 suggest that the disinfection rate for BNEF at a concentration of 10 ppm is actually faster at a pH level of 8 as compared to the disinfection rate at a pH level of 7. Therefore, the effect of increased pH on the efficacy of BNEF in water applications appears to be favorable. Increased disinfection rate with higher pH is also found in β-bromo-β-nitrostyrene, a commercially successful analog of BNEF.

Table 3 shows the minimum inhibitory concentration of BNEF against various microorganisms, in addition to Pseudomonas aeruginosa and Bacillus megaterium discussed earlier, encountered in various water treatment applications. The data show the compound has a broad spectrum in its activity. All of the minimum inhibitory concentration determinations were made in a nutrient-rich growth medium in the following manner. Each organism was inoculated into a nutrient-rich medium that supported its growth. Various concentrations of a solution of the antimicrobial in THFA (90 THFA, 10% antimicrobial) were dosed into the inoculated growth medium: inoculated media were also dosed only with THFA (no antimicrobial) to serve as controls. Two concentrations of antimicrobial were recorded for each organism after growth in the controls was visible (the lowest concentration that prevented growth and the highest concentration that allowed growth): the MIC is reported as the range between these concentrations.

The data in Table 3 are significant because they show an extraordinary spectrum of activity for this antimicrobial; broad-spectrum of activity is clearly an advantage for applications of industrial antimicrobials.

**Table 3**

| Minimum inhibitory concentration against various microrganisms. | | |
|---|---|---|
| Organism | Type of Microorganism | MIC(ppm) |
| Stachylococcus aureus | Bacterium | 3.0-7.6 |
| Xanthomonas orunii | Bacterium | 2.0-5.0 |
| Xanthomonas palaroonii | Bacterium | 1.0-2.0 |
| Xanthomonas poinsettiacola | Bacterium | 0.5-1.0 |
| Erwina chvsanthemi | Bacterium | 0.5-1.0 |
| Erwina anvlovora | Bacterium | ^{<}0.5 |
| Erwina caratovora | Bacterium | 0.5-1.0 |
| Aorobacterium tumefaciens | Bacterium | 0.5-1.0 |
| Photobacterium phosohoreum | Bacterium | 1.0-5.0 |
| Desulfovibro desulfuricans | Bacterium | ^{<}2.0 |
| Alternaria solani | Fungus | ^{<}1.0 |
| Asoergillus niger | Fungus | ^{<}1.0 |
| Saccharomyces sp. | Fungus (yeast) | ^{<}3.0 |
| Trichoderma virde | Fungus | ^{<}5.0 |
| Rhizoctonia sp. | Fungus | ^{<}1.0 |
| Chlorella pyrenoidosa | Algae | 4-16 |

The following analogues of 2-(2-bromo-2-nitroethenyl)-furan listed in Table 4, have been synthesized and tested for antimicrobial efficacy. All of these compounds are active in the low ppm range against Pseudomonas aeruginosa. These compounds were also tested in the pulp and paper ASTM efficacy test and shown to be active at low levels. None of the compounds tested appear to be more active than 2-(2-bromo-2-nitroethenyl)-furan.

**Table 4**

| Minimum inhibitory concentration of 2-(2-bromo-2-nitroethenyl)-furan analogues against Pseudomonas aeruginosa. | |
|---|---|
| Compound | MIC* Against Pseudomonas aeruinosa (ppm) |
| 5-nitro-2-(2-bromo-2-nitroethenyl)-furan | 8-16 |
| 5-bromo-2-(2-bromo-2-nitroetnenyl)-furan | 10-20 |
| 5-methyl-2-(2-bromo-2-nitroethenyl)-furan | 21-42 |
| 2-(2-bromo-2-nitroethenyl)-thiophene | 21-42 |

| | |
|---|---|
| *MIC - Minimum Inhibitory Concentration | |

## Claims

1. An antimicrobial composition comprising 2-(2-bromo-2-nitroethenyl)-furan and a carrier in liquid form. wherein the carrier is tetrahydrofuran, tetrahydrofurfuryl alcohol, N-methylpyrrolidone, or dimethylformamide.

2. A composition as claimed in claim 1 wherein the carrier is a tetrahydrofurfuryl alcohol.

3. A method for inhibiting microbial organisms selected from of algae, fungi and bacteria in an aqueous media which comprises adding an antimicrobially effective amount in the range of 0.5 ppm to 300 ppm of 2-(2-bromo-2-nitroethenyl)-furan, a broad spectrum antimicrobial agent, thereto.

4. A method as claimed in claim 3 wherein the bacterium is selected from Gram negative and Gram positive bacteria.

5. A method as claimed in claim 4 wherein the Gram negative bacteria is chosen from Pseudomonas and pseudomonads, Xanthomonous, Erwinia, Agrobacterium, Photobacterium, and Desulfovibrio.

6. A method as claimed in claim 4 wherein the Gram positive bacteria is chosen from Bacillus, Staphylococcus, Streptococcus, and Clostridium.

7. A method as claimed in claim 3 wherein the algae is Chlorella pyrenoidosa, Schizothrix, Anabaena, and Chlamydomonas.

8. A method as claimed in claim 3 wherein the fungus is chosen from Aspergillus, Trichomonas alternaria, Trichoderma, Saccharomyces, and Rhizoctonia.

9. A method as claimed in claim 3 wherein the aqueous media is selected from pulp and papermaking process flows, cooling water, metal working fluids, air-washers, oilfield injection water and drilling minds, acrylic latex paint emulsions, adhesives and coatings, swimming pools and spas, and cosmetics.

## Patentansprüche

1. Antimikrobielles Mittel, umfassend 2-(2-Brom-2-nitro-ethenyl)-furan und einen Träger in flüssiger Form, wobei der Träger Tetrahydrofuran, Tetrahydrofurfurylalkohol, N-Methylpyrrolidon oder Dimethylformamid ist.

2. Mittel nach Anspruch 1, wobei der Träger ein Tetrahydrofurfurylalkohol ist.

3. Verfahren zur Hemmung mikrobieller Organismen, ausgewählt aus Algen, Pilzen und Bakterien, in einem wäßrigen Medium, umfassend die Zugabe einer antimikrobiell wirksamen Menge im Bereich von 0,5 ppm bis 300 ppm von 2-(2-Brom-2-nitroethenyl)-furan, einem antimikrobiellen Mittel mit breitem Spektrum.

4. Verfahren nach Anspruch 3, wobei das Bakterium ausgewählt ist aus gramnegativen und grampositiven Bakterien.

5. Verfahren nach Anspruch 4, wobei die gramnegativen Bakterien ausgewählt sind aus Pseudomonas und Pseudomonaden, Xanthomonous, Erwinia, Agrobacterium, Photobacterium und Desulfovibrio.

6. Verfahren nach Anspruch 4, wobei die grampositiven Bakterien ausgewählt sind aus Bacillus, Staphylococcus, Streptococcus und Clostridium.

7. Verfahren nach Anspruch 3, wobei die Algen Chlorella pyrenoidosa, Schizothrix, Anabaena und Clamydomonas sind.

8. Verfahren nach Anspruch 3, wobei der Pilz ausgewählt ist aus Aspergillus, Trichomonas alternaria, Trichoderma, Saccharomyces, und Rhizoctonia.

9. Verfahren nach Anspruch 3, wobei das wäßrige Medium ausgewählt ist aus bei Pulpe- und Papierherstellungsverfahren anfallenden Flüssigkeiten, Kühlwasser, Metallverarbeitungsflüssigkeiten, Abluftwäschern, Ölfeldeinpreßwasser und Bohrschlamm, Acryllatexfarbemulsionen, Klebemitteln und Beschichtungen, Schwimmbädern und Mineralquellen und Kosmetika.

## Revendications

1. Composition antimicrobienne comprenant le 2-(2-bromo-2-nitroéthényl)-furanne et a support sous la forme liquide, dans laquelle le support est le tétrahydrofuranne, l'alcool tétrahydrofurfurylique, la N-méthylpyrrolidone ou le diméthylformamide.

2. Composition selon la revendication 1 dans laquelle le support est l'alcool tétrahydrofurfurylique.

3. Méthode pour inhiber les organismes microbiens choisis parmi les algues, les champignons et les bactéries dans un milieu aqueux comprenant l'addition d'une quantité efficace en tant qu'antimicrobien, dans l'intervalle allant de 0,5 à 300 ppm, de 2-(2-bromo-2-nitroéthényl)-furanne, agent antimicrobien à large spectre.

4. Méthode selon la revendication 3, dans laquelle la bactérie est sélectionnée dans le groupe des bactéries Gram négatif et Gram positif

5. Méthode selon la revendication 4, dans laquelle les bactéries Gram négatif sont choisies parmi *Pseudomonas* et *pseudomonades*, *Xanthomonous*, *Erwinia, Agrobacterium*, *Photobacterium* et *Desulfovibrio*.

6. Méthode selon la revendication 4, dans laquelle les bactéries Gram positif sont choisies parmi *Bacillus, Staphylococcus, Streptococcus* et *Clostridium*.

7. Méthode selon la revendication 3, dans laquelle les algues sont C*hlorella pyrenoidosa, Schizothrix, Anabaena* et *Chlamydomonas.*

8. Méthode selon la revendication 3, dans laquelle les champignons sont choisis parmi *Aspergillus, Trichomonas alternaria, Trichoderma, Saccharomyces,* et *Rhizoctonia.*

9. Méthode selon la revendication 3, dans laquelle le milieu aqueux est sélectionné dans les courants de procédé de la fabrication de la cellulose et du papier, l'eau de refroidissement, les fluides de traitement des métaux, les dispositifs de lavage d'air, l'eau d'injection et les boues de forage en exploitation pétrolière, les émulsions de peinture acrylique au latex, les adhésifs et revêtements, les piscines et spas et les cosmétiques.
